# EUROPEAN PATENT APPLICATION

(11) **EP 4 537 856 A1**
(43) Date of publication of application: **16.04.2025**
(21) Application number: 23819129.0
(22) Date of filing: 06.06.2023
(51) Int. Cl.: A61K 48/00, A61K 35/761, A61P 27/02, A61K 47/12, A61K 47/26, A61K 47/18

(54) **ADENO-ASSOCIATED VIRUS PHARMACEUTICAL COMPOSITION AND USE THEREOF**

(30) Priority: 07.06.2022 CN 202210633900
(71) Applicant: Chengdu Origen Biotechnology Co., Ltd., Chengdu, Sichuan 610037 (CN)
(72) Inventor: KE, Xiao, Chengdu, Sichuan 610036 (CN); ZHENG, Qiang, Chengdu, Sichuan 610036 (CN); JIANG, Hao, Chengdu, Sichuan 610036 (CN); QIN, Yingfei, Chengdu, Sichuan 610036 (CN); LUO, Junbo, Chengdu, Sichuan 610036 (CN)
(74) Representative: reuteler & cie SA
(86) International application number: PCT/CN2023/098693
(87) International publication number: WO 2023/236964

(57) **Abstract**

Provided is a pharmaceutical composition, the pharmaceutical composition comprising a recombinant adeno-associated virus (AAV), a buffer solution, an amino acid, a stabilizer, and a nonionic surfactant. The pharmaceutical composition has good stability.

## Description

### Field

The invention relates to the field of biotechnology, in particular to an adeno-associated virus pharmaceutical composition and a method thereof.

### Background

Adeno-associated virus (AAV) belongs to Parvoviridae and Dependoviruses. Recombinant adeno-associated viruses (rAAV) carrying therapeutic genes are widely used in various fields, such as gene transduction, gene therapy, vaccination, and oncolytic therapy, with many advantages including low pathogenicity, wide range of infected tissues, wide range of host cells (can infect and be expressed in proliferating and non-proliferating cells), low immunogenicity, long expression time of exogenous genes in vivo, and no integration into the host cell genome, so that it has been widely used in experimental and clinical research.

In the development of rAAV products, in addition to the safety and efficacy of the virus itself, it is also necessary to consider the stability of the product formulation, such as facing time and possible environmental changes (temperature, stress, light, etc.) during manufacturing, transportation, storage, and application. Therefore, in order to ensure the stability of product quality, development and improvement of the formulation are required.

### Summary

The purpose of the invention is to provide a stable pharmaceutical composition comprising recombinant adeno-associated virus (AAV).

In particular, the invention, on one hand, provides a pharmaceutical composition comprising a recombinant adeno-associated virus (AAV) and a buffer, an amino acid, a stabilizer, a nonionic surfactant.

In some embodiments, the buffer of the invention is a citrate buffer. In some preferred embodiments, the citrate is a sodium citrate, such as a sodium citrate monovalent, bivalent, or trivalent salt, preferably a trivalent salt.

In some embodiments, the amino acid of the invention is selected from aspartic acid, arginine, glycine, histidine, proline; more preferably, the amino acid is aspartic acid, proline, arginine.

In some embodiments, the stabilizer of the invention is selected from sucrose, trehalose, mannitol, lactose, galactose, glucose, maltose. In some preferred embodiments, the stabilizer of the invention is selected from sucrose, trehalose, mannitol. In some more preferred embodiments, the stabilizer of the invention is selected from sucrose or trehalose.

In some embodiments, the nonionic surfactant of the invention is selected from poloxamer 188, polysorbate 20, polysorbate 80, polyethylene glycol-hydroxystearate (HS15), tocopherol polyethylene glycol succinate (TPGS). In some preferred embodiments, the nonionic surfactant of the invention is selected from poloxamer 188.

In some embodiments, concentration of buffer in the pharmaceutical composition of the invention is 1 mM to 200 mM; concentration of amino acid is 5 mM to 200 mM; concentration of stabilizer is 1% to 20wt %; concentration of nonionic surfactant is 0.001% to 0.1wt%.

In some embodiments, concentration of buffer in the pharmaceutical composition of the invention is 1 mM to 100 mM; concentration of amino acid is 10 mM to 100 mM; concentration of stabilizer is 1% to 20wt %; concentration of nonionic surfactant is 0.001% to 0.1wt%.

In some embodiments, concentration of buffer in the pharmaceutical composition of the invention is 10 mM to 100 mM; concentration of amino acid is 20 mM to 150 mM; concentration of stabilizer is 2.5% to 10wt %; concentration of nonionic surfactant is 0.001% to 0.05wt%.

In some embodiments, concentration of buffer in the pharmaceutical composition of the invention is 10 mM to 80 mM; concentration of amino acid is 10 mM to 50 mM; concentration of stabilizer is 2.5% to 10wt %; concentration of nonionic surfactant is 0.001% to 0.05wt%.

In some embodiments, concentration of buffer in the pharmaceutical composition of the invention is 20 mM to 100 mM; concentration of amino acid is 40 mM to 150 mM; concentration of stabilizer is 3% to 10wt %; concentration of nonionic surfactant is 0.001% to 0.05wt%.

In some specific embodiments, the pharmaceutical composition of the invention comprises 40 mM buffer; 40 mM amino acid; 5%wt stabilizer; 0.001%wt nonionic surfactant.

In some specific embodiments, the pharmaceutical composition of the invention comprises 40 mM sodium citrate; 40 mM aspartic
In some specific embodiments, the pharmaceutical composition comprises 40 mM sodium citrate; 5%wt sucrose; 0.001%wt P188; 40 mM aspartic acid.

In some specific embodiments, the pharmaceutical composition comprises 40 mM sodium citrate; 5%wt sucrose; 0.001%wt P188; 40 mM arginine.

In some specific embodiments, the pharmaceutical composition comprises 40 mM sodium citrate; 5%wt sucrose; 0.001%wt P188; 40 mM proline.

In some specific embodiments, the pharmaceutical composition comprises 20 mM sodium citrate; 10%wt trehalose; 0.001%wt P188; 40 mM aspartic acid.

In some specific embodiments, the pharmaceutical composition comprises 100 mM sodium citrate; 3%wt sucrose; 0.001%wt P188; 80 mM aspartic acid.

In some specific embodiments, the pharmaceutical composition comprises 40 mM sodium citrate; 5%wt sucrose; 0.001%wt P188; 150 mM aspartic acid.

In some embodiments, the pharmaceutical composition of the invention further comprises MgCl₂. The concentration of magnesium chloride is 0.1 to 10 mM, preferably 1 mM to 5 mM.

In some embodiments, pH of the pharmaceutical composition of the invention is 5.5 to 8.0. In some preferred embodiments, pH of the pharmaceutical composition of the invention is 6.0 to 8.0. In some preferred embodiments, pH of the pharmaceutical composition of the invention is 5.5 to 7.5. In some preferred embodiments, pH of the pharmaceutical composition of the invention is 5.5 to 6.5. In some more preferred embodiments, pH of the pharmaceutical composition of the invention is 6.0±0.2. The pharmaceutical composition can be adjusted to the desired end-point pH by using sodium hydroxide or hydrochloric acid as a pH adjuster.

In some embodiments, the recombinant adeno-associated virus capsid protein serotype of the invention is selected from AAV1, AAV2, AAV3, AAV4, AAV5, AAV6, AAV7, AAV8, AAV9, AAV10, AAV11 or variants thereof. In some preferred embodiments, the recombinant adeno-associated virus capsid protein serotype of the invention is selected from AAV8 or a variant thereof.

In some embodiments, the recombinant adeno-associated virus of the invention comprises a coding sequence encoding a VEGF antagonist comprising at least one selected from the following groups:
a) a fusion protein comprising the amino acid sequence of SEQ ID NO: 1;
b) a fusion protein comprising the amino acid sequence of SEQ ID NO: 2;
c) with the antibody heavy chain variable region sequence of SEQ ID NO: 3 and the antibody light chain variable region of SEQ ID NO: 4;
d) with the antibody heavy chain variable region sequence of SEQ ID NO: 5 and the antibody light chain variable region sequence of SEQ ID NO: 6; preferably, a single-chain antibody comprising the amino acid sequence of SEQ ID NO: 7;
or an antibody or a protein comprises sequence with at least 80% identity to the above antibody or protein.

In some embodiments, recombinant adeno-associated virus of the invention comprises:
(i) a rAAV capsid protein, wherein the capsid protein is an AAV8 capsid protein or a variant thereof; and
(ii) a polynucleotide expression cassette, of which the order from 5 'to 3' comprising:
   (a) an AAV 5'ITR;
   (b) a CBA promoter;
   (c) a Kozak sequence;
   (d) a nucleic acid encoding an anti-VEGF antagonist, wherein the nucleic acid has a nucleotide sequence of SEQ ID NO:8;
   (e) a rabbit-globin polyadenylation signal;
   (f) an AAV 3'ITR.

In some specific embodiments, the polynucleotide expression cassette of the invention has a nucleotide sequence of SEQ ID NO:9.

In some embodiments, the pharmaceutical composition of the invention comprises recombinant adeno-associated virus (AAV) at a genomic concentration of about 1*10⁷vg/ml to 1*10¹⁴vg/ml. In some preferred embodiments, genome concentration of the recombinant adeno-associated virus (AAV) of the invention is approximately 1*10⁹vg/ml to 1*10¹³vg/ml; preferably 1*10⁹vg/ml to 1*10¹²vg/ml; preferably 1*10¹⁰vg/ml to 1*10¹³vg/ml.

In some preferred specific embodiments, genome concentration of the recombinant adeno-associated virus (AAV) in the invention is approximately 1 × 10⁷, 1.5 x 10⁷, 2 x 10⁷, 2.5 x 10⁷, 3 x 10⁷, 3.5 x 10⁷, 4× 10⁷, 4.5 x 10⁷, 5 × 10⁷, 5.5 x 10⁷, 6 × 10⁷, 6.5 x 10⁷, 7x 10⁷, 7.5 x 10⁷, 8 x 10⁷, 8.5 × 10⁷, 9 × 10⁷, 9.5 x 10⁷, 1 × 10⁸, 1.5 x 10⁸, 2 × 10⁸, 2.5 x 10⁸, 3 x 10⁸, 3.5 x 10⁸, 4 × 10⁸, 4.5 x 10⁸, 5 x 10⁸, 5.5 x 10⁸, 6 x 10⁸, 6.5 x 10⁸, 7 x 10⁸, 7.5 x 10⁸, 8 x 10⁸, 8.5 x 10⁸, 9 x 10⁸, 9.5 x 10⁸, 1 × 10⁹, 1.5 x 10⁹, 2 × 10⁹, 2.5 x 10⁹, 3 × 10⁹, 3.5 x 10⁹, 4 × 10⁹, 4.5 x 10⁹, 5 × 10⁹, 5.5 x 10⁹, 6 x 10⁹, 6.5 x 10⁹, 7 x 10⁹, 7.5 x 10⁹, 8 x 10⁹, 8.5 x 10⁹, 9 × 10⁹, 9.5 x 10⁹, 1 × 10¹⁰, 1.5 x 10¹⁰, 2 x 10¹⁰, 2.5 x 10¹⁰, 3 x 10¹⁰, 3.5 × 10¹⁰, 4 × 10¹⁰, 4.5 × 10¹⁰, 5 x 10¹⁰, 5.5 x 10¹⁰, 6 x 10¹⁰, 6.5 x 10¹⁰, 7 x 10¹⁰, 7.5 x 10¹⁰, 8 × 10¹⁰, 8.5 x 10¹⁰, 9 x 10¹⁰, 9.5 x 10¹⁰, 1 × 10¹¹, 1.5 x 10¹¹, 2 × 10¹¹, 2.5 x 10¹¹, 3 x 10¹¹, 3.5 x 10¹¹, 4 × 10¹¹, 4.5 × 10¹¹, 5 × 10¹¹, 5.5 × 10¹¹, 6 × 10¹¹, 6.5 × 10¹¹, 7 × 10¹¹, 7.5 × 10¹¹, 8 × 10¹¹, 8.5 × 10¹¹, 9 × 10¹¹, 9.5 × 10¹¹, 1 × 10¹², 1.5 × 10¹², 2 × 10¹², 2.5 × 10¹², 3 × 10¹², 3.5 × 10¹², 4 × 10¹², 4.5 × 10¹², 5 × 10¹², 5.5 x 10¹², 6 × 10¹², 6.5 x 10¹², 7 × 10¹², 7.5 x 10¹², 8 x 10¹², 8.5 x 10¹², 9 x 10¹², 9.5 × 10¹², 1 × 10¹³, 1.5 × 10¹³, 2 × 10¹³, 2.5 × 10¹³, 3 × 10¹³, 3.5 × 10¹³, 4 × 10¹³, 4.5 x 10¹³, 5 x 10¹³, 5.5 × 10¹³, 6 x 10¹³, 6.5 x 10¹³, 7 × 10¹³, 7.5 x 10⁸, 8x 10¹³, 8.5 × 10¹³, 9 × 10¹³, 9.5 x 10¹³, 1 × 10¹⁴ (all in vg/ml).

In some embodiments, the pharmaceutical composition of the invention is an intravitreal injection formulation, a subretinal injection formulation, an intrachoroidal injection formulation, an intravenous injection formulation, an intratumoral injection formulation or an intramuscular injection formulation. In some preferred embodiments, the pharmaceutical composition is an intravitreal injection formulation, a subretinal injection formulation or an intrachoroidal injection formulation.

In some embodiments, the pharmaceutical composition of the invention is a liquid composition or a pharmaceutical composition obtained by freeze-drying the liquid composition.

In some embodiments, the pharmaceutical composition of the invention is stored in a unit dose container. In some specific embodiments, the unit dose container is a vial or a syringe. In some preferred embodiments, the vial is a glass vial; in other preferred embodiments, the syringe is a pre-filled syringe.

Another aspect of the invention is to provide use of the pharmaceutical composition according to any one of the preceding claims in preparation of a drug for treating a disease related to VEGF.

In some embodiments, the VEGF-related disease is an ocular neovascularization.

In some preferred embodiments, the eye disease of the invention is selected from age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy and diabetic retinal oedema, macular oedema secondary to retinal vein occlusion, polypoidal choroidal vasculopathy, wet age-related macular degeneration with very low vision, choroidal neovascularization secondary to pathological myopia, neovascular glaucoma, iris neovascularization disease, retinopathy of prematurity.

Another aspect of the invention is to provide a method for treating a disease comprising administering a therapeutically effective amount of the recombinant adeno-associated viral drug composition in the invention to a patient/subject in need.

In some embodiments, the pharmaceutical composition in the invention is administered by subcutaneous injection, intramuscular injection, intravenous injection, intratumoral injection, intravitreal injection, suprachoroidal injection or subretinal injection. In some preferred embodiments, the pharmaceutical composition in the invention is administered by suprachoroidal or subretinal injection.

In some embodiments, the VEGF-related disease is an ocular disease.

In some preferred embodiments, the eye disease of the invention is selected from age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy, and diabetic retinal oedema, macular oedema secondary to retinal vein occlusion, polypoidal choroidal vasculopathy, wet age-related macular degeneration with very low vision, choroidal neovascularization secondary to pathological myopia, neovascular glaucoma, iris neovascularization disease, retinopathy of prematurity.

### Definitions

In order to facilitate the understanding of the invention, certain technical and scientific terms are specifically defined below. Unless expressly defined otherwise herein, all other technical and scientific terms used herein have meanings commonly understood by one of average skill in the art.

"Pharmaceutical composition" means it comprises one or more of the recombinant adeno-associated viruses (rAAV) described herein, together with other components such as a physiological/pharmaceutic vector and excipient. The purpose of the pharmaceutical composition is to facilitate the administration of drug to an organism, be beneficial to the absorption and/or expression of the active ingredient leading to biological activity. In this disclosure, "pharmaceutical composition" and "formulation" are not mutually exclusive.

As used herein, the term "AAV" refers to both naturally occurring adeno-associated virus and recombinant form of adeno-associated virus (rAAV) and includes mutant form of AAV. The term AAV further includes but is not limited to AAV type 1, AAV type 2, AAV type 3, AAV type 4, AAV type 5, AAV type 6, AAV type 7, AAV type 8, AAV type 9, AAV. Type 10, avian AAV, bovine AAV, canine AAV, equine AAV, sheep AAV, primate AAV, and non-primate AAV. In some embodiments, AAV is AAV8.

Pharmaceutically acceptable buffer is well known in the art and includes, but is not limited to, inorganic acid such as phosphate (sodium or potassium), bicarbonate, etc; organic acid such as citrate, acetate, succinate, etc; acidic buffer such as acetic acid, phosphoric acid, hydrochloric acid, carbonic acid, succinic acid, citric acid, histidine hydrochloric acid, malic acid, etc; alkaline buffer, such as sodium hydroxide, Tris, HEPES, etc.

"citrate" buffer is a buffer that includes citrate ions. Examples of citrate buffer include sodium citrate, potassium citrate, calcium citrate, magnesium citrate, etc. The preferred citrate buffer is sodium citrate.

Amino acid is well known in the art and includes natural and unnatural amino acid (synthetic amino acid). Examples such as: alanine (Ala); Valine (Val); Leucine (Leu); Isoleucine (Ile); Proline (Pro); Phenylalanine (Phe); Tryptophan (Trp); Methionine (Met); Glycine (Gly); Serine (Ser); Threonine (Thr); Cysteine (Cys); Tyrosine (Tyr); Asparagine (Asn); Glutamine (Gln); Selenocysteine (Sec); Pyrro lysine (Pyl); Lysine (Lys); Arginine (Arg); Histidine (His); Aspartic acid (Asp); Glutamic acid (Glu), etc.

It has been found that adding more than one sugar and/or sugar alcohol as a stabilizer at proper level (for example, between about 1% and about 10%) contributes to the stability of liquid formulation and/or freeze-dried formulation. Any sugar may be used as a stabilizer for use in the pharmaceutical composition of the invention, non-limiting examples include: monosaccharides, disaccharides or polysaccharides, or water-soluble glucans, including, for example, fructose, glucose, mannose, sorbose, xylose, maltose, lactose, sucrose, glucan, trehalose, amylopectin, dextrin, cyclodextrin, soluble starch, hydroxyethyl starch, and carboxymethyl cellulose. Sugar alcohol is defined as a hydrocarbon having about 4 to about 8 carbon atoms and one hydroxyl group. Non-limiting examples of sugar alcohols that may be used in the pharmaceutical composition provided by the invention include: mannitol, sorbitol, inositol, galactitol, melampyrit, xylitol, and arabitol.

The primarily nonionic surfactant that can be used in the pharmaceutical compositions of the invention is known in the pharmaceutical field, which includes, but not limited to polysorbate 80(Tween 80; PS80), polysorbate 20(Tween 20; PS20) and various poloxamer or pluronics, including pluronic F-68 and BRIJ 35, or mixture thereof.

"Freeze-dried formulation" means a formulation or pharmaceutical composition obtained following a vacuum freeze-drying step undergone by a pharmaceutical composition in liquid or solution form or a formulation of liquid or solution.

In representative embodiments, the pharmaceutical composition of the invention has a physiologically compatible pH value. In representative aspects, pH of the pharmaceutical composition is from about 5.0 to about 9.0, from about 5.5 to about 8.0, from about 6.0 to about 8.0, from about 5.5 to about 7.5, from about 6.0 to about 7.5, from about 6.5 to about 7.5. In certain embodiments, pH of the formulation is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, about 8.5, about 8.6, about 8.7, about 8.8, about 8.9, or about 9.0. In representative aspects, pH of the pharmaceutical composition is about 7.0 or about 7.5. In certain embodiments, pH of the pharmaceutical composition is about 6.0±0.2. In certain embodiments, pH of the pharmaceutical composition is about 7.0±0.2. In certain embodiments, pH of the pharmaceutical composition is about 8.0±0.2.

As used herein, the term "about" indicates an approximate range of positive or negative 10% from a specified value. For example, the term "about 20%" includes a range of 18-22%. As used herein, "about" also includes this exact value. Thus, "about 20%" means "about 20%" and "20%".

Formulation or pharmaceutical composition of the invention comprise additional pharmaceutically acceptable ingredients. In representative respects, the formulation or pharmaceutical composition comprises any one or a combination of the following: acidifiers, anticoagulants, antibacterial preservatives, antioxidants, preservatives, bases, inorganic salts, etc.

An "effective amount" includes an amount sufficient to ameliorate or prevent a symptom or disorder of a medical condition. An effective amount also means an amount sufficient to permit or facilitate a diagnosis. The effective amount used for a particular subject or veterinary subject may vary depending on factors such as the condition to be treated, the general health of the subject, the method and dose of administration, and the severity of the side effects. The effective dose may be the maximum dose or dosing regimen that avoids significant side effects or toxic effects.

The terms "patient" and "subject" are used interchangeable, and in their normal sense, to refer to an organism, including human and non-human animals, suffering from or susceptible to a condition that can be prevented or treated by administering a composition of the present invention. Examples of subjects include, but are not limited to: human, chimpanzee, other ape and monkey specie; farm animal such as cattle, sheep, pig, goat, and horse; domestic mammals such as dog and cat; laboratory animal, including rodent such as mouse, rat, and guinea pig; bird including poultry, wild bird, and game bird such as chicken, turkey, and other quail, duck, goose, etc. The term does not indicate a specific age. Thus, adults, adolescents, and newborn individuals are all objects.

"Stable" or "substantially stable" may refer to the pharmaceutical composition maintaining its properties (e.g., pH, osmotic pressure, genomic titer, capsid protein purity, rAAV purity, activity, insoluble microparticles maintained stable) over a long storage period. For example, a composition that is stable for storage does not have significant impurities due to degradation of the composition over a long period of time and maintain high purity. For example, it may have impurities less than 10%, or degradation products less than 9%, or 8%, or 7%, or 6%, or 5%, or 4%, or 3%, or 2%, or 1% over a longer period of time. In some cases, a composition that is stable for storage have little or a very limited amount of insoluble particles of ≥10µm or ≥25µm or ≥50µm or ≥100µm or ≥150µm or ≥200µm or ≥250µm or ≥300µm or ≥350µm or ≥400µm or ≥450µm or ≥500µm or larger over a long period of time. In some cases, a composition that is stable in storage substantially maintain its activity over extended periods of time, for example, the composition maintains 100%, or more than 99%, or more than 98%, or more than 97%, or more than 96%, or more than 95%, or more than 94%, or more than 93%, or more than 92%, or more than 91%, or more than 85%, or more than 80%, or more than 75% of its activity. A long time period is a period of time such as more than 1 week, or more than 2 weeks, or more than 3 weeks, or more than 1 month, or more than 2 months, or more than 3 months, or more than 4 months, or more than 6 months, or more than 9 months, or more than 1 year, or more than 1.5 years (such as 18 months), or more than 2 years, or more than 2.5 years (such as 30 months), or 3 years or more, or more than 3.5 years (e.g. 42 months), or more than 4 years, or more than 4.5 years (e.g. 54 months), or more than 5 years. In some embodiments, the stored stable pharmaceutical composition is substantially stable over an extended period of time at ambient temperature, such as a temperature of 20 to 40°C, or 25 to 35°C, or 25 to 30°C. In some cases, the stored stable composition is substantially stable for an extended period of time at a temperature below the ambient temperature, such as 0 to 20°C, or 0 to 15°C, or 0 to 10°C, or 2 to 8 °C.

In some cases, the term "stable" or "substantially stable" may also refer to the thermal stability of the AAV in the pharmaceutical composition resistant to high temperatures or temperature changes. Two pathways are involved in the thermal denaturation of AAV: genome ejection and capsid disruption. Genome ejection of AAV capsids occurs at relatively low temperatures and DNA begins to escape from the intact capsid. AAV capsid disruption occurs when protein unfolding causes the viral capsid to lose its structural integrity and break. To monitor or evaluate the thermal stability of AAV, conventional experimental methods in this field such as Elisa, qPCR, AAV-ID, and high-throughput thermal stability analysis can be used. In some embodiments, the invention utilizes the Uncle (Unchained Labs) multifunctional protein stability analysis system to monitor the genome ejection and capsid destruction of AAV to evaluate the thermal stability of the protein. Tracking genome ejection by using DNA binding fluorescent dyes such as SYBR Gold (nucleic acid dyes are able to combine with nucleic acids ejected from the AAV and emit fluorescence, the more nucleic acids ejected, the stronger the fluorescence signal after binding), and determining the melting temperature (Tm) based on DNA release. A higher Tm temperature indicates better thermal stability of the AAV. In some cases, Tm of AAV in the pharmaceutical composition of the invention is increased by at least 0.5 °C, or at least 1°C, or at least 1.5 °C, or at least 2°C, or at least 2.5 °C, or at least 3°C, or at least 3.5 °C, or at least 4°C, or at least 4.5 °C, or at least 5°C or more relative to other compositions. The disruption of AAV capsid can be studied on Uncle by monitoring the intrinsic protein fluorescence of capsid proteins without dyes (when the protein is heated to a certain point, the protein unravels, the hydrophobic region is exposed, and the protein autofluorescence changes, so the protein structure can be determined by changes in the protein autofluorescence). The capsid protein of the AAV in the pharmaceutical composition in the invention undergoes little structural change.

### Brief Description of the Drawings

Fig. 1A shows SDS-PAGE results of capsid protein of the samples in Example 2 placed at room temperature for 0 day.
Fig. 1B shows SDS-PAGE results of capsid protein of the samples in Example 2 placed at room temperature for 3 days.
Fig. 2 shows thermal stability test results of the samples in Example 9.

### Detailed Description

The disclosure is further described combined with specific examples below. It can be understood that these examples are used only to illustrate the invention and are not intended to limit the scope of the disclosure.

### Example 1 Preparation of a recombinant adeno-associated viral vector (rAAV) stock solution

A recombinant plasmid containing the target protein gene described in the invention was constructed and cloned in *Escherichia coli* using conventional DNA recombination techniques and cloning techniques and the plasmid was subsequently used for the preparation of recombinant AAV virus.

Firstly, the polynucleotide expression cassette and recombinant plasmid encoding the target gene was constructed. Exemplary, the expression cassette or plasmid comprising, in 5' to 3' order: an AAV 5'ITR, a CBA promoter, a Kozak sequence, a nucleic acid of the coding sequence encoding anti-VEGF protein, a rabit-globin polyadenylation signal, an AAV 3'ITR."

The polynucleotide expression cassette and the recombinant plasmid have a coding sequence encoding the Conbercept fusion protein (target gene), wherein Conbercept has an amino acid sequence as described in SEQ ID NO:2. The recombinant adeno-associated viral vector (rAAV) was prepared by the three-plasmid co-transfection process known in the art. After the three plasmids (pAAV-Conbercept: pAAV 8-RC-Kan: pHelper-Kan) were transfected into HEK293 cells, the rAAV genome titer was detected for use.

### Example 2

Buffer preparation: appropriate amounts of sodium chloride, disodium hydrogen phosphate, sodium dihydrogen phosphate and poloxamer 188 were weighed respectively, the solvent was water, and the buffer solution was formulated to contain 180mM sodium chloride, 10 mM disodium hydrogen phosphate-sodium dihydrogen phosphate buffer, 0.001wt% poloxamer 188, and the pH value was adjusted about 7 with dilute hydrochloric acid or sodium hydroxide solution.

Sample solution preparation: an appropriate amount of the stock solution described in Example 1 was taken and injected into the dialysis card, and dialysis fluid exchange was performed according to the volume ratio of the formulation buffer to the stock solution ≥100 times. A total of 3 times of fluid exchanges were performed for 2 hours each time, and three different groups of formulations were obtained. In the final samples, concentration of disodium hydrogen phosphate-sodium dihydrogen phosphat buffer was 10 mM, concentration of sodium chloride was 180 mM, concentration of poloxamer 188 was 0.001wt%, pH was about 7, and concentration of rAAV was 1 x 10¹¹vg/ml.

The samples were placed at room temperature (for 3 days), and the purity of the rAAV capsid protein was used as an indicator to evaluate the stability of the samples by SDS-PAGE.

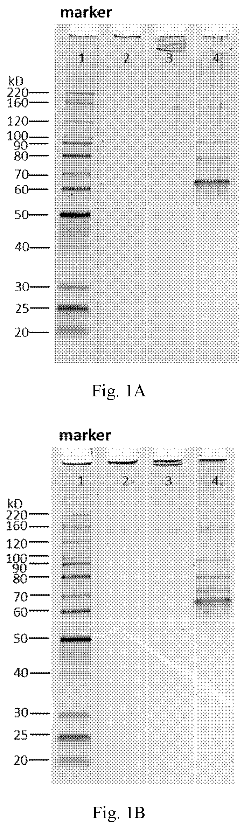

The results were shown in Fig. 1A and 1B. The samples were investigated at room temperature for 3 days (Figure 1B) and the results showed an obvious miscellaneous band at 70 kDa relative to the original sample (Figure 1A) (lane 4).

### Example 3

Buffer preparation: appropriate amount of glycine, sodium citrate, trehalose and poloxamer 188 were weighed respectively, the solvent was water, a buffer solution containing 40mM glycine, 40mM sodium citrate buffer, 5wt% trehalose and 0.001wt% poloxamer 188 was formulated, and the pH value was adjusted by dilute hydrochloric acid or sodium hydroxide solution to about 7.2.

Sample solution preparation: an appropriate amount of the stock solution as described in Example 1 was taken and injected into the dialysis card, and dialysis fluid exchange was performed according to the volume ratio of the formulation buffer to the stock solution ≥100 times. A total of 3 times of fluid exchanges were performed each 2 hours. The concentration of rAAV in the sample solution is 1 x 10¹¹vg/ml.

### Example 4

Buffer preparation: appropriate amounts of aspartic acid, sodium citrate, trehalose and poloxamer 188 were weighed respectively, the solvent was water, a buffer solution containing 40mM aspartic acid, 40mM sodium citrate buffer, 5wt% trehalose and 0.001wt% poloxamer 188 was formulated, and the pH value was adjusted about 7.2 with dilute hydrochloric acid or sodium hydroxide solution.

Sample solution preparation: another appropriate amount of the stock solution as described in Example 1 was taken and injected into the dialysis card, dialysis fluid exchange was performed according to the volume ratio of the formulation buffer to the stock solution ≥100 times. A total of 3 times of fluid exchanges were performed each 2 hours. The concentration of rAAV in the sample solution is 1 x 10¹¹vg/ml.

### Example 5

Buffer preparation: appropriate amounts of aspartic acid, sodium citrate, sucrose, and poloxamer 188 were weighed respectively, the solvent was water, a buffer solution containing 40mM aspartic acid, 40mM sodium citrate buffer, 5wt% sucrose, and 0.001wt% poloxamer 188 was formulated, and the pH value was adjusted about 6.0 with dilute hydrochloric acid or sodium hydroxide solution."

Sample solution preparation: another appropriate amount of the stock solution described in Example 1 was taken and injected into the dialysis card, and dialysis fluid exchange was performed according to the volume ratio of preparation buffer to stock solution ≥100 times. A total of 3 times of fluid exchanges were performed each 2 hours, and 5 groups of sample solutions were formulated. Concentrations of rAAV in the sample solutions were 1.0×10¹³ vg/mL, 5.0×10¹²vg/mL, 1.0×10¹²vg/mL, 1.0×10¹¹vg/mL and 1.0×10¹⁰vg/mL, respectively.

The long-term stability (≤-65 °C , 9 months) of the prepared 1.0×10¹³ vg/mL sample was investigated. The test indexes included pH, osmotic pressure, genome titer, capsid protein purity, rAAV purity, insoluble particles, etc., as shown in Table 1.

**Table 1**

| | **pH** | **Osmotic pressure (mOsmol/kg)** | **Genome titer (vg/mL)** | **Capsid protein purity (VP1+VP2+VP3)** | **rAAV purity** | **insoluble particles (number of particles/mL)** | | |
|---|---|---|---|---|---|---|---|---|
| | | | | | | **≥10 µm** | **≥25 µm** | **≥50 µm** |
| 0 months | 6.0 | 342 | 9.97 × 10¹² | 97.2% | 98.10% | 2 | 1 | 0 |
| 3 months | 6.0 | 345 | 1.10 × 10¹³ | 96.0% | 99.45% | 2 | 0 | 0 |
| 6 months | 6.1 | 349 | 9.03 × 10¹² | 96.8% | 97.78% | 3 | 0 | 0 |
| 9 months | 6.0 | 350 | 1.02 × 10¹³ | 96.1% | 98.48% | 1 | 0 | 0 |

### Example 6

The stock solution described in Example 1 was taken and formulated as rAAV formulation solutions according to the formulations in Table 2-1 and Table 2-2 below, wherein the concentrations of AAV were 1.0×10¹² vg/mL, respectively, and pH of the solutions were adjusted with dilute hydrochloric acid or sodium hydroxide solution.

**Table 2-1**

| | Example 6-1 | Example 6-2 | Example 6-3 | Example 6-4 | Example 6-5 | Example 6-6 | Example 6-7 | Example 6-8 |
|---|---|---|---|---|---|---|---|---|
| Sucrose | 5% | / | / | / | 4% | 5% | 5% | 5% |
| Aspartic acid | 40 mM | / | / | / | / | 40 mM | 40 mM | 40 mM |
| Sodium citrate | 40 mM | / | / | / | / | / | / | / |
| Tris | / | / | 20 mM | / | / | 40 mM | / | / |
| Na₂HPO₄.2H₂O | / | 10 mM | / | 8 mM | 8 mM | / | 10 mM | 1.47mM |
| NaH₂PO₄.2H₂O | / | 10 mM | / | / | / | / | 10 mM | / |
| KH₂PO₄ | / | / | / | 1.47mM | 1.47mM | / | / | 20 mM |
| NaCl | / | 180 mM | 200 mM | 140 mM | 100 mM | / | / | / |
| KCl | / | / | / | 2.7mM | 2.7mM | / | / | / |
| MgCl₂.6H₂O | 1 mM | / | 1 mM | / | / | 1 mM | 1 mM | 1 mM |
| P188 | 0.001% | 0.001% | 0.001% | 0.001% | 0.001% | 0.001% | 0.001% | 0.001% |
| pH value | 6.0 | 7.3 | 8.0 | 7.5 | 7.5 | 6.0 | 7.3 | 8.0 |

**Table 2-2**

| | Example 6-9 | Example 6-10 | Example 6-11 |
|---|---|---|---|
| Sucrose | 5% | 5% | / |
| Trehalose | | / | 5% |
| Aspartic acid | / | / | 40mM |
| Arginine | 40mM | / | / |
| Proline | / | 40mM | / |
| Sodium citrate | 40mM | 40mM | 40mM |
| MgCl₂.6H₂O | 1mM | 1mM | 1mM |
| P188 | 0.001% | 0.001% | 0.001% |
| pH value | 6.0 | 6.0 | 6.1 |

The thermal stability of rAAV in the above formulation solutions were investigated by protein thermostability (Tm) assay. In Tm assay, rAAV samples were treated with nucleic acid dye, and then the gene ejection from the sample at a certain temperature range (25° C-95 °C) was real-time detected by fluorescence, static light scattering (SLS), dynamic light scattering (DLS), etc. The specific operations were as follows: 20X SYBRTM GOLD nucleic acid dye was added to the above formulation samples, and 9 µl was added to the Uni tube, then real-time detection was performed by selecting the program "Tm,Tagg&Optional DLS" in the high-throughput multiparameter protein temperature analysis system (UNcle, UNCHAINED LABS). The parameters of the high-throughput multi-parameter protein temperature analysis system were set as follows: starting temperature 25°C, incubation time 180s, heating rate 0.5°C/min, termination temperature 95°C, UV 266 Opaque(0.00), Blue Laser open(1.00). The results of the samples are shown in Tables 3-1 and 3-2.

**Table 3-1**

| | Example 6-1 | Example 6-2 | Example 6-3 | Example 6-4 | Example 6-5 | Example 6-6 | Example 6-7 | Example 6-8 |
|---|---|---|---|---|---|---|---|---|
| Tm1 (°C) | 74.52 | 68.64 | 69.83 | 68.60 | 69.17 | 71.16 | 70.77 | 70.70 |

**Table 3-2**

| | Example 6-9 | Example 6-10 | Example 6-11 |
|---|---|---|---|
| Tm1 (°C) | 73.00 | 73.09 | 73.53 |

### Example 7

The stock solution described in Example 1 was taken and formulated as rAAV formulation solutions according to the formulations in Table 4 below, wherein the concentrations of AAV were 1.0×10¹² vg/mL, respectively, and pH of the solutions were adjusted with dilute hydrochloric acid or sodium hydroxide solution.

**Table 4**

| | Example 6-1 | Example 7-1 | Example 7-2 | Example 7-3 | Example 7-4 |
|---|---|---|---|---|---|
| Sucrose | 5% | 5% | 5% | 5% | 5% |
| Aspartic acid | 40mM | 40mM | 40mM | 40mM | 40mM |
| Sodium citrate | 40 mM | 40 mM | 40 mM | 40 mM | 40 mM |
| P188 | 0.001% | 0.01% | / | / | / |
| Tween 20 | / | / | 0.01% | / | / |
| HS 15 | / | / | / | 0.01% | / |
| TPGS | / | / | / | / | 0.01% |
| MgCl₂.6H₂O | 1 mM | 1 mM | 1 mM | 1 mM | 1 mM |
| pH value | 6.0 | 6.0 | 6.0 | 6.0 | 6.0 |

Tm assay was performed according to the method in Example 6. The results are shown in Table 5.

**Table 5**

| | Example 6-1 | Example 7-1 | Example 7-2 | Example 7-3 | Example 7-4 |
|---|---|---|---|---|---|
| Tm1 (°C) | 73.46 | 73.65 | 73.76 | 74.00 | 74.09 |

### Example 8

The stock solution described in Example 1 was taken and formulated as rAAV formulation solutions according to the formulations in Table 6 below, wherein concentrations of AAV were 1.0×10¹²vg/mL, respectively, and pH of the solutions were adjusted with dilute hydrochloric acid or sodium hydroxide solution.

**Table 6**

| | Example 6-1 | Example 8-1 | Example 8-2 |
|---|---|---|---|
| Sucrose | 5% | 5% | 5% |
| Aspartic acid | 40 mM | 40 mM | 40 mM |
| Sodium citrate | 40 mM | 40 mM | 40 mM |
| P188 | 0.001% | 0.001% | 0.001% |
| MgCl₂.6H₂O | 1 mM | 1 mM | 1 mM |
| pH value | 6.0 | 7.5 | 8.0 |

Tm assay was performed according to the method in Example 6. The results are shown in Table 7.

**Table 7**

| | Example 6-1 | Example 8-1 | Example 8-2 |
|---|---|---|---|
| Tm1 (°C) | 74.60 | 72.81 | 72.84 |

### Example 9

The stock solution described in Example 1 was taken and formulated as rAAV formulation solutions according to the formulations in Table 8 below, wherein the concentrations of AAV were 1.0×10¹² vg/mL, respectively, and pH of the solutions were adjusted with dilute hydrochloric acid or sodium hydroxide solution.

**Table 8**

| | Example 9-1 | Exampl e 9-2 | Example 9-3 | Exampl e 9-4 | Example 9-5 | Example 9-6 | Example 9-7 | Exampl e 9-8 | Example 9-9 | Example 6-1 |
|---|---|---|---|---|---|---|---|---|---|---|
| Sucrose | / | 1% | 5% | / | / | / | / | 5% | / | 5% |
| Trehalose | / | / | / | / | / | / | / | / | / | / |
| Sodium citrate | 10mM | 15mM | / | / | / | / | / | 40mM | 40mM | 40mM |
| NaAc | / | / | / | 40mM | / | / | / | / | / | |
| KCl | / | / | / | / | 2.7 mM | / | / | / | / | |
| KH₂PO₄ | / | / | / | / | 1.47 mM | / | / | / | / | |
| NaCl | 150mM | / | 100mM | / | 137mM | 200mM | 200mM | / | / | |
| Na₂HPO₄ ·H₂O | / | / | / | / | 8.1 mM | / | / | / | / | |
| Tris | / | / | / | / | / | 20mM | / | / | / | |
| HEPES | / | / | / | / | / | / | 20mM | / | / | |
| Aspartic acid | / | / | / | 40mM | 40mM | 40mM | 40mM | / | 40mM | 40mM |
| MgCl₂. 6H₂O | / | / | / | 1mM | 1mM | 1mM | 1mM | 1mM | 1mM | 1mM |
| P188 | / | / | / | 0.001% | 0.001% | 0.001% | 0.001% | 0.001% | 0.001% | 0.001% |
| Histidine | / | / | 10mM | / | / | / | / | / | / | |
| Arginine | / | | 50mM | / | / | / | / | / | / | |
| Glycine | | 10mM | | | | | | | | |
| Mannitol | / | 2% | / | / | / | / | / | / | / | |
| Tween 80 | 0.003% | 0.005% | 0.005% | / | / | / | / | / | / | |
| pH | 7.0 | 7.0 | 7.0 | 6.0 | 6.0 | 6.0 | 6.0 | 6.0 | 6.0 | 6.0 |

The thermal stability of rAAV in the above preparation solutions were investigated by isothermal stability assays. The samples were kept at a specific temperature for a period of time (e.g., 18h, 24h), and the capsid protein structural changes (e.g., aggregation/denaturation) of the sample rAAVs were detected in real time by fluorescence, static light scattering (SLS), dynamic light scattering (DLS), etc. The specific operations are as follows: 9 µl of the above preparation samples were added to the Uni tube, and the assays were performed by selecting the selection program "Isothermal" from "Isothermal Toolbox" in the high-throughput multi-parameter protein temperature analysis system (UNcle, UNCHAINED LABS). The parameters of the high throughput multi-parameter protein temperature analysis system were as follows: temperature 42°C, constant temperature time: 5min, investigation time: 18h, UV 266 Filter1(0.50), Blue Laser Filter3(0.25). The relative BCM (300-430 nm) response values were used to observe the changes in the thermal stability of proteins in different formulations. By stacking the sample curves that need to be compared with time, observing the time and speed of the rise of the curve can know the time and speed of the sample aggregation/denaturation, and then evaluate the stability of the sample.

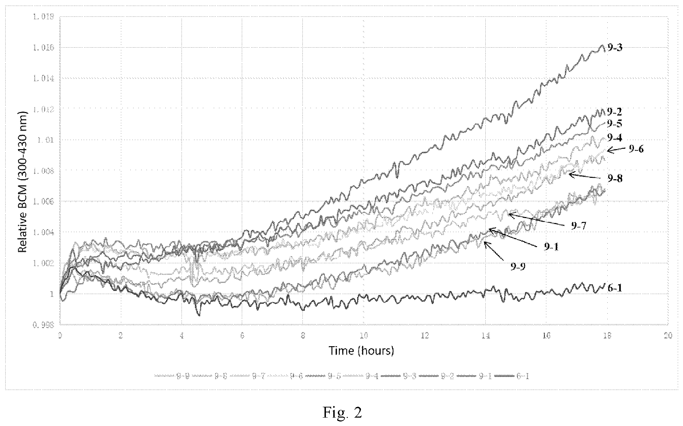

The results are shown in Fig. 2. As can be seen from Fig. 2, the curve of the sample of Example 6-1 changed gently from 0 to 18h, and the relative BCM index fluctuant at 1±0.002, indicating that there was basically no aggregation or denaturation of the sample during the incubation process at 42°C, and the sample had good stability; The sample curves of Examples 9-1, 9-7 and 9-9 have relatively larger changes, and the relative BCM refers to a fluctuation in the range of 1-1.007; The sample curves of Examples 9-2, 9-4, 9-5, 9-6 and 9-8 change more greatly, and the relative BCM refers to the fluctuation in the range of 1-1.012; The sample curve of Example 9-3 has the largest change, and the relative BCM refers to a fluctuation in the range of 1-1.016.

### Example 10

The stock solution described in Example 1 was taken and formulated as rAAV formulation solutions according to the formulations in Table 9 below, wherein the concentrations of AAV were 1.0×10¹² vg/mL, respectively, and pH of the solutions were adjusted with dilute hydrochloric acid or sodium hydroxide solution.

**Table 9**

| | Example 10-1 | Example 10-2 | Example 10-3 |
|---|---|---|---|
| Sucrose | 5% | 5% | / |
| Trehalose | / | / | 5% |
| Aspartic acid | / | / | 40mM |
| Arginine | 40mM | / | / |
| Proline | / | 40mM | / |
| Sodium citrate | 40mM | 40mM | 40mM |
| MgCl_{2·}6H₂O | 1mM | 1mM | 1mM |
| P188 | 0.001% | 0.001% | 0.001% |
| pH value | 6.0 | 6.0 | 6.0 |

Isothermal stability assays were performed according to the method described in Example 9, and the results showed that the curves of the formulation samples of Example 10-1, 10-2, and 10-3 changed gently from 0 to 18h, with a relative BCM value of 1±0.002, maintaining good thermal stability.

### Example 11

The stock solution described in Example 1 was taken and formulated as a rAAV formulation comprising 40mM sodium citrate; 5%wt sucrose; 0.001%wt P188; 40mM aspartic acid, in which concentration of AAV was 1.0×10¹² vg/mL, and the solution was adjusted to pH 6.0±0.2 with dilute hydrochloric acid or sodium hydroxide solution."

The isothermal stability assay was performed according to the method described in Example 9. The results showed that the curve of the rAAV formulation sample changed gently within 0-18 h, and the relative BCM value was 1±0.002, which had good thermal stability.

### Example 12

The stock solution described in Example 1 was taken and formulated as a rAAV formulation comprising 20mM sodium citrate; 10%wt sucrose; 0.001%wt P188; 40mM aspartic acid; 1mM magnesium chloride hexahydrate, in which concentration of AAV was 1.0×10¹² vg/mL, and the solution was adjusted to pH 6.0±0.2 with dilute hydrochloric acid or sodium hydroxide solution.

The isothermal stability assay was performed according to the method described in Example 9. The results showed that the curve of the rAAV formulation sample changed gently within 0-18h, and the relative BCM value was 1±0.002, which had good thermal stability.

### Example 13

The stock solution described in Example 1 was taken and formulated as a rAAV formulation comprising 100mM sodium citrate; 3%wt sucrose; 0.001%wt P188; 80mM aspartic acid; 1mM magnesium chloride hexahydrate, in which the concentration of AAV was 1.0×10¹² vg/mL, and the solution was adjusted to pH 6.0±0.2 with dilute hydrochloric acid or sodium hydroxide solution.

The isothermal stability assay was performed according to the method described in Example 9. The results showed that the curve of the rAAV formulation sample changed gently within 0-18h, and the relative BCM value was 1±0.002, which had good thermal stability.

### Example 14

The stock solution described in Example 1 was taken and formulated as a rAAV formulation comprising 40mM sodium citrate; 5%wt sucrose; 0.001%wt P188; 150mM aspartic acid; 1mM magnesium chloride hexahydrate, in which the concentration of AAV was 1.0×10¹² vg/mL, respectively, and the solution was adjusted to pH 6.0±0.2 with dilute hydrochloric acid or sodium hydroxide solution.

The isothermal stability assay was performed according to the method described in Example 9. The results showed that the curve of the rAAV formulation sample changed gently within 0-18h, and the relative BCM value was 1±0.002, which had good thermal stability.

### Example 15

The stock solution described in Example 1 was taken and formulated as a rAAV formulation comprising 40mM sodium citrate; 5%wt sucrose; 0.001%wt P188; 40mM aspartic acid; 1mM magnesium chloride hexahydrate, in which the concentration of AAV was 1.0×10¹¹ vg/mL, respectively, and the solution was adjusted to pH 6.0±0.2 with dilute hydrochloric acid or sodium hydroxide solution.

The isothermal stability assay was performed according to the method described in Example 9. The results showed that the curve of the rAAV formulation sample changed gently within 0-18h, and the relative BCM value was 1±0.002, which had good thermal stability.

### Example 16

The stock solution described in Example 1 was taken and formulated as a rAAV formulation comprising 40mM sodium citrate; 5%wt sucrose; 0.001%wt P188; 40mM aspartic acid, 1mM magnesium chloride hexahydrate, in which the concentration of AAV was 1.0×10¹⁰vg/mL, respectively, and the solution was adjusted to pH 6.0±0.2 with dilute hydrochloric acid or sodium hydroxide solution.

The isothermal stability assay was performed according to the method described in Example 9. The results showed that the curve of the rAAV formulation sample changed gently within 0-18h, and the relative BCM value was 1±0.002, which had good thermal stability.

### Example 17

The stock solution described in Example 1 was taken and formulated as a rAAV formulation comprising 40mM sodium citrate; 5%wt sucrose; 0.001%wt P188; 40mM aspartic acid; 1mM magnesium chloride hexahydrate, in which the concentration of AAV was 1.0×10¹³vg/mL, respectively, and the solution was adjusted to pH 6.0±0.2 with dilute hydrochloric acid or sodium hydroxide solution."

The isothermal stability assay was performed according to the method described in Example 9. The results showed that the curve of the rAAV formulation sample changed gently within 0-18h, and the relative BCM value was 1±0.002, which had good thermal stability.

## Claims

1. A pharmaceutical composition **characterized in that** the pharmaceutical composition comprises a recombinant adeno-associated virus and a buffer, an amino acid, a stabilizer, a nonionic surfactant, with the buffer being a citrate buffer.

2. The pharmaceutical composition according to claim 1, wherein the citrate is a sodium citrate; more preferably, the sodium citrate is a sodium citrate trivalent salt.

3. The pharmaceutical composition according to any one of the preceding claims, wherein the amino acid is selected from aspartic acid, arginine, glycine, histidine, proline; more preferably, the amino acid is aspartic acid, proline, arginine.

4. The pharmaceutical composition according to any one of the preceding claims, wherein the stabilizer is selected from sucrose, trehalose, mannitol, lactose, galactose, glucose, maltose; preferably is sucrose or trehalose.

5. The pharmaceutical composition according to any one of the preceding claims, wherein the nonionic surfactant is selected from poloxamer 188, polysorbate 20, polysorbate 80, HS15, TPGS; preferably is poloxamer 188.

6. The pharmaceutical composition according to any one of the preceding claims, wherein in the pharmaceutical composition, the concentration of buffer is 1 mM to 200 mM; the concentration of amino acids is 5 mM to 200 mM; the concentration of stabilizer is 1% to 20 wt%; the concentration of nonionic surfactant is 0.001% to 0.1wt%.

7. The pharmaceutical composition according to any one of the preceding claims, wherein in the pharmaceutical composition, the concentration of buffer is 10 mM to 100 mM; the concentration of amino acid is 20 mM to 150 mM; the concentration of stabilizer is 2.5% to 10wt%; the concentration of nonionic surfactant is 0.001% to 0.05wt%.

8. The pharmaceutical composition according to any one of the preceding claims, wherein in the pharmaceutical composition, the concentration of buffer is 20 mM to 100 mM; the concentration of amino acids is 40 mM to 150 mM; the concentration of stabilizer is 3% to 10wt%; the concentration of nonionic surfactant is 0.001% to 0.05wt%.

9. The pharmaceutical composition according to any one of the preceding claims, wherein the pharmaceutical composition comprises 40 mM buffer, 40 mM amino acids, 5%wt stabilizer, 0.001%wt nonionic surfactant.

10. The pharmaceutical composition according to any one of the preceding claims, wherein the pharmaceutical composition comprises 40 mM sodium citrate, 40 mM aspartic acid, 5%wt sucrose, 0.001%wt P188.

11. The pharmaceutical composition according to any one of the preceding claims, wherein the pharmaceutical composition further comprises MgCl₂.

12. The pharmaceutical composition according to any one of the preceding claims, wherein pH of the pharmaceutical composition is 5.5 to 8.0; preferably, pH of the pharmaceutical composition is 6.0 to 8.0; preferably, pH is 5.5 to 6.5; preferably, pH is 6.0±0.2.

13. The pharmaceutical composition according to any one of the preceding claims, wherein the genome concentration of the recombinant adeno-associated virus in the pharmaceutical composition is about 1*10⁷vg/ml to 1*10¹⁴vg/ml; preferably, the genome concentration of the recombinant adeno-associated virus is about 1*10⁹vg/ml to 1*10¹³vg/ml; more preferably, the genome concentration of the recombinant adeno-associated virus is about 1*10⁹vg/ml to 1*10¹²vg/ml.

14. The pharmaceutical composition according to any one of the preceding claims, wherein the recombinant adeno-associated virus capsid protein serotype is selected from AAV1, AAV2, AAV3, AAV4, AAV5, AAV6, AAV7, AAV8, AAV9, AAV10, AAV11, or a variant thereof.

15. The pharmaceutical composition according to any one of the preceding claims, wherein the recombinant adeno-associated virus capsid protein serotype is selected from AAV8 or a variant thereof.

16. The pharmaceutical composition according to any one of the preceding claims, wherein the recombinant adeno-associated virus comprises a coding sequence encoding a VEGF antagonist comprising at least one selected from the following group:
a) a fusion protein comprising the amino acid sequence of SEQ ID NO: 1;
b) a fusion protein comprising the amino acid sequence of SEQ ID NO: 2;
c) with the antibody heavy chain variable region sequence of SEQ ID NO: 3 and the antibody light chain variable region sequence of SEQ ID NO: 4;
d) with the antibody heavy chain variable region sequence of SEQ ID NO: 5 and the antibody light chain variable region sequence of SEQ ID NO: 6; preferably a single-chain antibody comprising the amino acid sequence of SEQ ID NO: 7; or an antibody or a protein comprises sequence with at least 80% identity to the above antibody or protein.

17. The pharmaceutical composition according to any one of the preceding claims, wherein the recombinant adeno-associated virus comprises:
(i) a rAAV capsid protein, wherein the capsid protein is an AAV8 capsid protein or a variant thereof; and
(ii) a polynucleotide expression cassette, of which the order from 5 'to 3' comprising:
(a) an AAV 5'ITR;
(b) a CBA promoter;
(c) a Kozak sequence;
(d) a nucleic acid encoding an anti-VEGF antagonist, wherein the nucleic acid has a nucleotide sequence of SEQ **ID** NO:8;
(e) a rabbit-globin polyadenylation signal;
(f) an AAV 3'ITR.

18. The pharmaceutical composition according to any one of the preceding claims, wherein the
polynucleotide expression cassette has a nucleotide sequence of SEQ ID NO:9.

19. The pharmaceutical composition according to any one of the preceding claims, wherein the pharmaceutical composition is an intravitreal injection formulation, a subretinal injection formulation, an intrachoroidal injection formulation, an intravenous injection formulation, an intratumoral injection formulation or an intramuscular injection formulation; preferably, the pharmaceutical composition is an intravitreal injection formulation, a subretinal injection formulation, an intrachoroidal injection formulation.

20. The pharmaceutical composition according to any one of the preceding claims, wherein the pharmaceutical composition is a liquid formulation.

21. The pharmaceutical composition according to any one of the preceding claims, wherein the pharmaceutical composition is a freeze-dried formulation.

22. The pharmaceutical composition according to any of the preceding claims, wherein the pharmaceutical composition is stored in a unit dose container.

23. The pharmaceutical composition of claim 22, wherein the unit dose container is a vial or a syringe.

24. The pharmaceutical composition of claim 22, wherein the vial is a glass vial.

25. The pharmaceutical composition of claim 22, wherein the syringe is a pre-filled syringe.

26. Use of the pharmaceutical composition according to any one of the preceding claims in preparation of a drug for treating a VEGF-related disease.

27. A method for treating a VEGF-related disease, comprising administering a therapeutically effective amount of the pharmaceutical composition of any one of claims 1-25 to a patient/subject in need.

28. The use according to claim 26 or the method according to claim 27, wherein the VEGF-related disease is an ocular neovascularization disease.

29. The use or the method according to claim 28, wherein the ocular neovascularization disease is selected from age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy and diabetic retinal oedema, macular oedema secondary to retinal vein occlusion, polypoid choroidal vasculopathy, wet age-related macular degeneration with very low vision, choroidal neovascularization secondary to pathological myopia, neovascular glaucoma, iris neovascularization disease, retinopathy of prematurity.
